# EUROPEAN PATENT SPECIFICATION

(11) **EP 4 127 103 B1**
(45) Date of publication and mention of the grant of the patent: **03.06.2026**
(21) Application number: 21727790.4
(22) Date of filing: 17.05.2021
(51) Int. Cl.: C10G 7/00, B01D 3/14, B01D 1/28, C07C 7/04

(54) **HEAT INTEGRATION VIA HEAT PUMP ON A BOTTOM DIVIDING WALL COLUMN**
WÄRMEINTEGRATION ÜBER WÄRMEPUMPE AN EINER BODENTRENNWANDSÄULE
INTÉGRATION DE CHALEUR PAR L'INTERMÉDIAIRE D'UNE POMPE À CHALEUR SUR UNE COLONNE DE PAROI DE SÉPARATION INFÉRIEURE

(30) Priority: 20.05.2020 US 202063027440 P
(43) Date of publication of application: 08.02.2023
(73) Proprietor: Sulzer Management AG, 8401 Winterthur (CH)
(72) Inventor: SILVA, Michael, Houston, Texas 77004 (US); DE, Saurav, Katy, Texas 77494 (US); MARTIN, Gary R., Grapevine, Texas 76051 (US)
(74) Representative: IPS Irsch AG
(86) International application number: PCT/EP2021/062926
(87) International publication number: WO 2021/233801

(56) References cited:
- LONG NGUYEN VAN DUC ET AL: "Design and optimization of heat integrated dividing wall columns for improved debutanizing and deisobutanizing fractionation of NGL", KOREAN JOURNAL OF CHEMICAL ENGINEERING, vol. 30, no. 2, 1 February 2013 (2013-02-01), US, KR, pages 286 - 294, XP055831863, ISSN: 0256-1115, Retrieved from the Internet <URL:http://citeseerx.ist.psu.edu/viewdoc/download?doi=10.1.1.918.5192&rep=rep1&type=pdf> [retrieved on 20210812], DOI: 10.1007/s11814-012-0149-2
- AURANGZEB MD ET AL: "Vapor recompression with interreboiler in a ternary dividing wall column: Improving energy efficiency and savings, and economic performance", APPLIED THERMAL ENGINEERING, vol. 147, 1 January 2019 (2019-01-01), GB, pages 1009 - 1023, XP055831897, ISSN: 1359-4311, Retrieved from the Internet <URL:https://www.sciencedirect.com/science/article/pii/S1359431118347999/pdfft?md5=da0691fd8843f863d20df0266b1e8ca6&pid=1-s2.0-S1359431118347999-main.pdf> [retrieved on 20210812], DOI: 10.1016/j.applthermaleng.2018.11.008

## Description

### FIELD OF THE INVENTION

The present invention relates to a dividing wall column system particularly for the use in a de-isopentanizer (DIP) or de-isobutanizer (DIB) application. The present invention further relates to a method for producing an iso-C₄-hydrocarbon composition from a hydrocarbon feed.

### BACKGROUND OF THE INVENTION

DIPs and DIBs are typically part of a Natural Gas Liquid (NGL) fractionation plant. NGL fractionation configurations are typically made up of a series of columns that distill the lighter component in each successive column. For example, if fractionating hydrocarbon molecules from C₁-C₅₊, the following columns may be used: demethanizer column, deethanizer column, depropanizer column, and a debutanizer column. Additional columns such as DIPs or DIBs may be added to further fractionate a particular component into its paraffinic and iso-paraffinic components. More specifically, in a typical NGL fractionation train, a debutanizer column produces a distillate with a mixture of n-butane and isobutane and this distillate is then fed to a deisobutanizer column, in which i-C₄ and n-C₄-hydrocarbon fractions are separated. With so many columns and reboilers in the configuration, there are strategic spots to include heat integration to help in the overall utility usage of the plant. Long and Lee disclose in "Design and optimization of heat integrated dividing wall columns for improved debutanizing fractionation of NGL" in Korean J. Chem. Eng., volume 30, 2013, pages 286 to 294 a dividing wall column and a multi-effect prefractionator arrangement including a heat pump in the bottom of the dividing wall column. Aurangzeb and Jana describe in "Vapor recompression with interreboiler in a ternary dividing wall column: Improviding energy efficiency and savings, and economic performance" in Applied Thermal Engineering, volume 147, 2019, pages 1009 to 1023 ternary dividing wall columns being connected with a vapor recompression heat pump system in conjunction with an interreboiler.

The object underlying the present invention was to provide a dividing wall column system particularly for the use in a de-isopentanizer or de-isobutanizer application and to provide a method for producing an iso-C₄-hydrocarbon composition from a hydrocarbon feed, wherein the method and system are energy efficient, i.e. the system requires comparable low investment costs and the method has comparable low operational costs, but with both, the system and the method nevertheless having a very high separation efficiency.

### SUMMARY OF THE INVENTION

In accordance with the present invention, this object is satisfied by providing a dividing wall column system being particularly suitable for the use as a de-isopentanizer or a de-isobutanizer comprising: i) a dividing wall column comprising a dividing wall positioned in the bottom section of the dividing wall column to divide the bottom section of the dividing wall column into a first side and a second side, ii) a first reboiler outside of the dividing wall column and in fluid communication with the first side of the bottom section of the dividing wall column, iii) a second reboiler outside of the dividing wall column and in fluid communication with the second side of the bottom section of the dividing wall column and iv) a heat pump in fluid communication with the dividing wall column and the second reboiler, with the heat pump being configured so as to compress a first portion of the overhead product from the dividing wall column, a second condenser in fluid communication with the dividing wall column and configured to receive a second portion of the overhead product from the dividing wall column; and a first condenser in fluid communication with the second reboiler.

It has been found by the inventors of the present invention that by using a dividing wall column comprising a dividing wall positioned in the bottom section of the dividing wall column to divide the bottom section of the dividing wall column into a first side and a second side, i.e. by using a bottom dividing wall column, which is in fluid communication with a heat pump, wherein the heat pump is also in fluid communication with the second reboiler so that a first portion of the overhead product (or light product respectively) of the dividing wall column is compressed in the heat pump and then fed to the second reboiler in order to heat at least a portion of the bottom product withdrawn from the second side of the bottom section of the bottom dividing wall column, the energy consumption of the dividing wall column system, in particular when used as a de-isopentanizer or a de-isobutanizer, can be drastically decreased, but nevertheless an excellently high separation efficiency is obtained. The vapor generated in the first reboiler is used to strip the feed on the first side of the bottom section defined by the dividing wall of C₄-hydrocarbon material, which produces a C₅₊-hydrocarbon product on that side of the bottom section of the dividing wall column. Conversely, the vapor generated in the second reboiler is used to strip i-C₄-hydrocarbons to the top of the dividing wall column. All in all, according to the present invention, the overhead product or vapor, respectively, produced by the dividing wall column is used to heat the second side or C₄-reboiler, respectively. Only a portion of the overhead vapor stream is used and preferably the bottom dividing wall column further includes one overhead condenser and an accumulator for reflux of a portion of the overhead product. As such, the balance of overhead vapor will continue to a conventional overhead condenser before being collected in the preferred overhead accumulator arranged downstream of the second reboiler along with the condensed vapor that was used to heat the reboiler. The dividing wall column is typically operated at a pressure of less than 1 MPag. Therefore, the overhead vapor from this column is close to typical cooling water supply temperature ranges. As such, by condensing this vapor stream in the reboiler, cooling water requirements and heating medium requirements will be reduced. Thus, the present invention is a heat integration scheme that uses a heat pump to compress the overhead vapor obtained in the dividing wall column so as to increase its temperature to heat the second reboiler of the dividing wall column.

Heat pump means in the present invention any device being able to compress the light product, i.e. the overheads product, of the bottom dividing wall column. Thus, the heat pump may comprise one or more compressors and may consist of one or more compressors. For instance, the heat pump may comprise or consist of one or more turbofans.

The second reboiler may be any kind of reboiler or heat exchanger, respectively. Examples therefore are plate and shell reboilers, compabloc heat exchangers, shell and tube reboilers and the like. For instance, the second reboiler is a shell and tube reboiler and the bottom product of the second side of the dividing wall column is transported through the shell side of the second reboiler, whereas the portion of the overhead product of the dividing wall column, which has been compressed in the heat pump, is transported through the tube of the second reboiler.

In accordance with the present invention, the dividing wall column is a bottom dividing wall column, i.e. a dividing wall column comprising a dividing wall positioned in the bottom section of the dividing wall column to divide the bottom section of the dividing wall column into a first side and a second side. The dividing wall extends preferably from the bottom of the dividing wall column over a part of the height of the dividing wall column at least essentially vertically upwards. Essentially vertically upwards may mean that the angle between the dividing wall and the length axis of the dividing wall column is at most 40°, preferably at most 20°, more preferably at most 10° and most preferably 0°, wherein the height of the dividing wall column is the straight distance between the top and the bottom of the dividing wall column. Such a bottom dividing wall column comprises two independent stripping sections on either side of the dividing wall (namely the first and second sides of the bottom section) and a common rectifying section in the top section above the dividing wall.

It is preferred that the dividing wall of the dividing wall column extends from the bottom of the dividing wall column, seen from the bottom to the top of the dividing wall column, over 10 to 80%, more preferably over 10 to 70%, yet more preferably over 20 to 70%, even more preferably over 30 to 70% and most preferably over 40 to 60% of the height of the dividing wall column. The bottom section is that part of the dividing wall column, in which the dividing wall is located and defines the first and second sides, whereas the top section is the part of the dividing wall column above the dividing wall.

The dividing wall column may comprise at least one internal element selected from the group consisting of trays, structured packings, random packings and arbitrary combinations of two or more of the aforementioned elements, in order to improve its mass and heat transfer efficiency. In addition, where necessary distributors and collectors may be included above and below the one or more internal elements.

In accordance with the present patent application the dividing wall column system further comprises a first condenser, which is in fluid communication with the second reboiler, i.e. the dividing wall column system further comprises a line or conduit, respectively, between the second reboiler and the first condenser.

In accordance with a further embodiment of the present invention, the second reboiler is fluidly coupled to the dividing wall column to feed-reflux to the second side of the dividing wall column. This means that preferably the second reboiler comprises a line connecting the second side of the bottom section of the dividing wall column with the second reboiler so as to feed during the operation bottom product from the second side of the bottom section of the dividing wall column into the second reboiler and a further line connecting the second reboiler with the second side of the bottom section of the dividing wall column so as to feed during the operation bottom product from the second reboiler back into the second side of the bottom section of the dividing wall.

Preferably, also the first reboiler is fluidly coupled to the dividing wall column to feed-reflux to the first side of the dividing wall column, i.e. the first reboiler comprises a line connecting the first side of the bottom section of the dividing wall column with the first reboiler so as to feed during the operation bottom product from the first side of the bottom section of the dividing wall column into the first reboiler and a further line connecting the first reboiler with the first side of the bottom section of the dividing wall column so as to feed during the operation bottom product from the first reboiler back into the first side of the bottom section of the dividing wall.

According to the present invention, the dividing wall column system further comprises a second condenser in fluid communication with the dividing wall column and configured to receive a second portion of the overhead product from the dividing wall column. In other words, the dividing wall column system further comprises a second condenser which is connected with the overhead of the dividing wall column by a line or conduit, respectively. This second portion of the overhead product is not led over the heat pump

In a further development of the idea of the present patent application it is suggested that the dividing wall column system further comprises an accumulator in fluid communication with the first and second condensers. Thus, preferably a first line or conduit, respectively connects the accumulator with the first condenser and a second line or conduit, respectively connects the accumulator with the second condenser.

Preferably, in the aforementioned embodiment of the present invention, the accumulator is in fluid communication with the dividing wall column to feed-reflux to the dividing wall column, i.e. a recirculation line connects the accumulator with the dividing wall column. Preferably, the recirculation line is connected with the top section or overhead of the dividing wall column.

In accordance with a further embodiment of the present invention, downstream of the first condenser a third condenser is arranged in fluid communication with the first condenser.

According to a further aspect, the present invention relates to a method of producing an i-C₄-hydrocarbon product, which is performed in the aforementioned dividing wall column system.

More specifically, the method comprises the steps of:
i) introducing a feed containing hydrocarbons to the dividing wall column of the aforementioned dividing wall column system;
ii) feeding, from the first side of the dividing wall column, the bottom product comprising C₅₊-hydrocarbons to the first reboiler;
iii) feeding, from the second side of the dividing wall column, the bottom product comprising n-C₄-hydrocarbons to the second reboiler;
iv) feeding a first portion of the overhead product from the dividing wall column to the heat pump and compressing the first portion of the overhead product therein; and
v) feeding the compressed overhead product from the heat pump to the second reboiler to exchange heat between the compressed overhead product and the bottom product comprising n-C₄-hydrocarbons, and
feeding the compressed overhead product from the second reboiler to a first condenser and condensing it therein, wherein the dividing wall column system further comprises a second condenser and a second portion of the overhead product is fed from the dividing wall column to the second condenser.

Preferably, a portion of the bottom product comprising n-C₄-hydrocarbons is fed as reflux stream from the second reboiler to the second side of the bottom section of the dividing wall column, whereas the other part of the bottom product comprising n-C₄-hydrocarbons is withdrawn from the dividing wall column system.

Likewise, it is preferred that a portion of the bottom product comprising C₅₊-hydrocarbons is fed as reflux stream from the first reboiler to the first side of the bottom section of the dividing wall column, whereas the other part of the bottom product comprising C₅₊-hydrocarbons is withdrawn from the dividing wall column system.

In accordance with the present patent application the method further comprises the step of feeding the compressed overhead product

from the second reboiler, after it has heat exchanged in the second reboiler with the bottom product comprising n-C₄-hydrocarbons, to a first condenser being arranged downstream of the second reboiler and condensing the bottom product comprising n-C₄-hydrocarbons in the first condenser.

It is preferred in the aforementioned embodiment that the dividing wall column system further comprises an accumulator and that the overhead product is fed from the first condenser to the accumulator. Alternatively, between the first condenser and the accumulator a further condensor may be arranged so as to further cool or condense the bottom product comprising n-C₄-hydrocarbons therein.

In accordance with a further embodiment of the present invention, the method further comprises the step of leading a reflux stream from the accumulator to the dividing wall column and in particular into the top section or overhead of the dividing wall column.

In accordance with the present patent application the dividing wall column system further comprises a second condenser and preferably - in addition to the first portion of the overhead product being fed into the heat pump - a second portion of the overhead product is fed from the dividing wall column to the second condenser so as to at least partially condense the overhead product therein.

In a further development of the idea of the present patent application it is suggested that in the aforementioned embodiment the second portion of the overhead product, which has been preferably at least partially condensed in the second condenser, is fed to the accumulator.

The method may further comprise the step of producing an i-C4-hydrocarbon feed in the accumulator.

### BRIEF DESCRIPTION OF THE DRAWINGS

Subsequently, the present invention is described with reference to an illustrative, but not limiting drawing, in which:

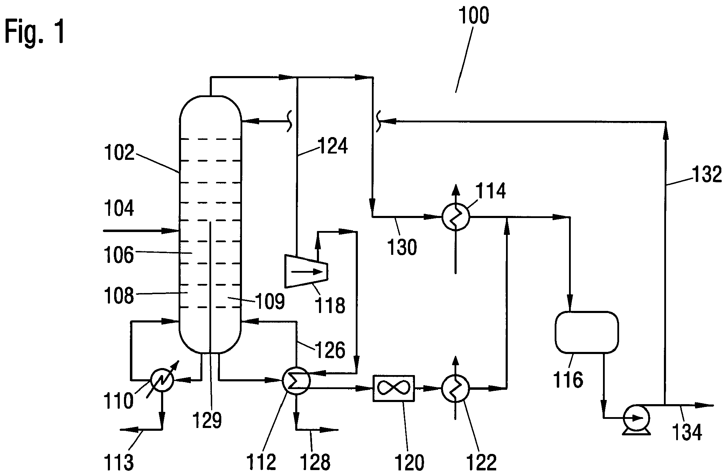

Fig. 1 illustrates a bottom dividing wall column system with a heat pump in accordance with an embodiment of the present invention.

### DESCRIPTION

Various aspects will now be described more fully with reference to the accompanying drawing. The disclosure may, however, be embodied in many different forms and should not be construed as limited to the aspects set forth herein.

Fig. 1 illustrates a bottom dividing wall column system 100 according to an embodiment of the present invention. The system 100 includes a dividing wall column 102 that receives a feed 104 that includes hydrocarbons. The dividing wall column 102 includes a bottom dividing wall 106 that divides the bottom section of the dividing wall column 102 into two sides 108, 109. The bottom dividing wall 106 extends vertically from the bottom of the dividing wall column 102 upwardly over about 50% of the height of the dividing wall column 102. Furthermore, the dividing wall column system 100 includes a first reboiler 110 and a second reboiler 112 that are fluidly coupled to sides 108, 109 of the bottom sections of the dividing wall column 102 respectively. The second reboiler 112 is embodied as shell and tube reboiler. The first reboiler 110 strips the feed on the first side 108 of the dividing wall 106 of C₄-hydrocarbons and produces a C₅₊-product stream on that first side 108 of the dividing wall 106, which is withdrawn from the dividing column system 100 via the C₅₊-hydrocarbon product line 113. The second reboiler 112 strips i-C₄-hydrocarbons and produces a n-C₄-hydrocarbon product stream. In addition, the bottom dividing wall column system 100 comprises a compressor 118 (or heat pump, respectively), a first condenser 120, a second condenser 114, a third condenser 122 and an (overhead) accumulator 116.

The dividing wall column 102 outputs an overhead product (or light product, respectively) at the top of the dividing wall column. A first portion of the overhead product is fed via line 124 to the compressor 118, where it is compressed. The compressed overhead product is then fed into the tube side of the second reboiler 112, whereas the bottom product withdrawn from the bottom section of the dividing wall column 102 is fed into the shell of the second reboiler 112 so that a heat exchange between the warmer compressed overhead product and the colder bottom product is effected, which leads to an at least partial vaporization of the n-C₄-hydrocarbon bottom product obtained in the second side 109 of the bottom section of the dividing wall column 102. While a portion of the n-C₄-hydrocarbon bottom product is refluxed from the shell of the second reboiler 112 via the reflux line 126 back into the bottom section of the dividing wall column 102, the other portion of the n-C₄-hydrocarbon bottom product is withdrawn from the dividing column system 100 via the n-C₄-hydrocarbon product line 128. Alternatively, a part of or all of the n-C₄-hydrocarbon product stream may be withdrawn from the column sump 129 instead of from the reboiler 112. The compressed overhead product is fed from the tube side of the second reboiler 112 to the first condenser 120, from there to the third condenser 122 and from there into the accumulator 116.

The second portion of the overhead product is fed via line 130 directly, i.e. under bypassing the compressor 118, to the second condenser 114 and from there to the accumulator 116, in which it is combined with the first portion of the overhead product which has been led through the compressor 118 and through the second reboiler 112. From the accumulator 116, a portion of the i-C₄-hydrocarbon overhead product is recycled via reflux line 132 into the top section of the dividing wall column 102, whereas the other portion of the i-C₄-hydrocarbon overhead product is withdrawn from the dividing column system 100 via the i-C₄-hydrocarbon product line 134.

The dividing wall column 102 is typically operated at a pressure of less than 1 MPag. The light product (i.e. overhead vapor) from dividing wall column 102 is close to typical cooling water supply temperature ranges. As such, by condensing the light product in reboiler 112, cooling water requirements and heating medium requirements are reduced.

Benefits of bottom dividing wall column system 100 include:
- Reduction in overhead condensing duty, thereby requiring less cooling water supply.
- Reduction in hot medium requirement (typically hot oil/steam) for the n-C₄-reboiler.
- Savings in operating costs.
- Smaller footprint of the hot oil system due to a lower requirement of hot oil.
- Lower heater emissions due to lower hot oil requirement.

### Reference Numerals

- 100: (Bottom) dividing wall column system
- 102: (Bottom) dividing wall column
- 104: Feed
- 106: Bottom dividing wall
- 108: First side of the bottom section of the (bottom) dividing wall column
- 109: Second side of the bottom section of the (bottom) dividing wall column
- 110: First reboiler
- 112: Second reboiler
- 113: n-C₅₊-hydrocarbon product line
- 114: (Second) condenser
- 116: (Overhead) accumulator
- 118: Heat pump/compressor
- 120: (First) condenser
- 122: (Third) condenser
- 124: Overheads line to the compressor
- 126: Reflux line in bottom section of the dividing wall column
- 128: n-C₄-hydrocarbon product line
- 129: Column sump
- 130: Overheads line bypassing the compressor
- 132: Reflux line to top section of the dividing wall column
- 134: i-C₄-hydrocarbon product line 134

## Claims

1. A dividing wall column system (100) comprises:
a dividing wall column (102) comprising a dividing wall (106) positioned in the bottom section of the dividing wall column (102) to divide the bottom section of the dividing wall column (102) into a first side (108) and a second side (109);
a first reboiler (110) outside of the dividing wall column (102) and in fluid communication with the first side (108) of the bottom section of the dividing wall column (102);
a second reboiler (112) outside of the dividing wall column (102) and in fluid communication with the second side (109) of the bottom section of the dividing wall column (102);
a heat pump (118) in fluid communication with the dividing wall column (102) and the second reboiler (112) and configured to compress a first portion of the overhead product from the dividing wall column (102);
a second condenser (114) in fluid communication with the dividing wall column (102) and configured to receive a second portion of the overhead product from the dividing wall column (102); and
a first condenser (120) in fluid communication with the second reboiler (112).

2. The dividing wall column system (100) of claim 1, wherein the second reboiler (112) is fluidly coupled to the dividing wall column (102) to feed-reflux to the second side (109) of the dividing wall column (102) and preferably also the first reboiler (110) is fluidly coupled to the dividing wall column (102) to feed-reflux to the first side (108) of the dividing wall column (102).

3. The dividing wall column system (100) of claim 1, further comprising an accumulator (116) in fluid communication with the first and second condensers (120, 114).

4. The dividing wall column system (100) of claim 1 or 3, wherein the accumulator (116) is in fluid communication with the dividing wall column (102) to feed-reflux to the dividing wall column (102).

5. A method of producing an i-C₄-hydrocarbon product, the method comprising:
introducing a feed containing hydrocarbons to the dividing wall column (102) of the dividing wall column system (100) in accordance with any of claims 1 to 4;
feeding, from the first side (108) of the dividing wall column (102), the bottom product comprising C₅₊-hydrocarbons to the first reboiler (110);
feeding, from the second side (109) of the dividing wall column (102), the bottom product comprising n-C₄-hydrocarbons to the second reboiler (112);
feeding a first portion of the overhead product from the dividing wall column (102) to the heat pump (118) and compressing the first portion of the overhead product therein;
feeding the compressed overhead product from the heat pump (118) to the second reboiler (112) to exchange heat between the compressed overhead product and the bottom product comprising n-C₄-hydrocarbons, and
feeding the compressed overhead product from the second reboiler (112) to a first condenser (120) and condensing it therein,
wherein the dividing wall column system (100) further comprises a second condenser (114) and a second portion of the overhead product is fed from the dividing wall column (102) to the second condenser (114).

6. The method of claim 5, wherein a reflux stream is fed from the second reboiler (112) to the second side (109) of the bottom section of the dividing wall column (102).

7. The method of claim 5 or 6, wherein a reflux stream is fed from the first reboiler (110) to the first side (108) of the bottom section of the dividing wall column (102).

8. The method of claim 7, wherein the dividing wall column system (100) further comprises an accumulator (116) and the overhead product is fed from the first condenser (120) to the accumulator (116).

9. The method of claim 5, further comprising feeding the second portion of the overhead product to the accumulator (116).

10. The method of claim 9, further comprising producing an i-C4-hydrocarbon feed from the accumulator (116).

11. The method of claim 10, further comprising leading a reflux stream from the accumulator (116) to the dividing wall column (102).

## Patentansprüche

1. Trennwandsäulensystem (100), umfassend:
eine Trennwandsäule (102), umfassend eine Trennwand (106), die in dem Bodenabschnitt der Trennwandsäule (102) positioniert ist, um den Bodenabschnitt der Trennwandsäule (102) in eine erste Seite (108) und eine zweite Seite (109) zu unterteilen;
einen ersten Verdampfer (110) ausserhalb der Trennwandsäule (102) und in Fluidverbindung mit der ersten Seite (108) des Bodenabschnitts der Trennwandsäule (102);
einen zweiten Verdampfer (112) ausserhalb der Trennwandsäule (102) und in Fluidverbindung mit der zweiten Seite (109) des Bodenabschnitts der Trennwandsäule (102);
eine Wärmepumpe (118) in Fluidverbindung mit der Trennwandsäule (102) und dem zweiten Verdampfer (112) und konfiguriert, um einen ersten Teil des Kopfprodukts aus der Trennwandsäule (102) zu komprimieren;
einen zweiten Kondensator (114) in Fluidverbindung mit der Trennwandsäule (102) und konfiguriert, um einen zweiten Teil des Kopfprodukts aus der Trennwandsäule (102) aufzunehmen; und
einen ersten Kondensator (120) in Fluidverbindung mit dem zweiten Verdampfer (112).

2. Trennwandsäulensystem (100) nach Anspruch 1, wobei der zweite Verdampfer (112) fluidisch mit der Trennwandsäule (102) gekoppelt ist, um der zweiten Seite (109) der Trennwandsäule (102) Rücklauf zuzuführen, und vorzugsweise auch der erste Verdampfer (110) fluidisch mit der Trennwandsäule (102) gekoppelt ist, um der ersten Seite (108) der Trennwandsäule (102) Rücklauf zuzuführen.

3. Trennwandsäulensystem (100) nach Anspruch 1, ferner umfassend einen Akkumulator (116) in Fluidverbindung mit dem ersten und dem zweiten Kondensator (120, 114).

4. Trennwandsäulensystem (100) nach Anspruch 1 oder 3, wobei der Akkumulator (116) in Fluidverbindung mit der Trennwandsäule (102) steht, um der Trennwandsäule (102) Rücklauf zuzuführen.

5. Verfahren zum Herstellen eines i-C₄-Kohlenwasserstoffprodukts, wobei das Verfahren umfasst:
Einleiten eines Kohlenwasserstoffe enthaltenden Einsatzmaterials in die Trennwandsäule (102) des Trennwandsäulensystems (100) nach einem der Ansprüche 1 bis 4;
Zuführen des Bodenprodukts, das C₅₊-Kohlenwasserstoffe umfasst, von der ersten Seite (108) der Trennwandsäule (102) zu dem ersten Verdampfer (110);
Zuführen des Bodenprodukts, das n-C₄-Kohlenwasserstoffe umfasst, von der zweiten Seite (109) der Trennwandsäule (102) zu dem zweiten Verdampfer (112);
Zuführen eines ersten Teils des Kopfprodukts aus der Trennwandsäule (102) zu der Wärmepumpe (118) und Komprimieren des ersten Teils des Kopfprodukts darin;
Zuführen des komprimierten Kopfprodukts aus der Wärmepumpe (118) zu dem zweiten Verdampfer (112), um Wärme zwischen dem komprimierten Kopfprodukt und dem Bodenprodukt, das n-C₄₋Kohlenwasserstoffe umfasst, auszutauschen, und
Zuführen des komprimierten Kopfprodukts aus dem zweiten Verdampfer (112) zu einem ersten Kondensator (120) und Kondensieren desselben darin,
wobei das Trennwandsäulensystem (100) ferner einen zweiten Kondensator (114) umfasst und ein zweiter Teil des Kopfprodukts aus der Trennwandsäule (102) dem zweiten Kondensator (114) zugeführt wird.

6. Verfahren nach Anspruch 5, wobei ein Rücklaufstrom aus dem zweiten Verdampfer (112) der zweiten Seite (109) des Bodenabschnitts der Trennwandsäule (102) zugeführt wird.

7. Verfahren nach Anspruch 5 oder 6, wobei ein Rücklaufstrom aus dem ersten Verdampfer (110) der ersten Seite (108) des Bodenabschnitts der Trennwandsäule (102) zugeführt wird.

8. Verfahren nach Anspruch 7, wobei das Trennwandsäulensystem (100) ferner einen Akkumulator (116) umfasst und das Kopfprodukt aus dem ersten Kondensator (120) dem Akkumulator (116) zugeführt wird.

9. Verfahren nach Anspruch 5, ferner umfassend Zuführen des zweiten Teils des Kopfprodukts zu dem Akkumulator (116).

10. Verfahren nach Anspruch 9, ferner umfassend die Herstellung einer i-C₄-Kohlenwasserstoffzufuhr aus dem Akkumulator (116).

11. Verfahren nach Anspruch 10, ferner umfassend Leiten eines Rücklaufstroms aus dem Akkumulator (116) zu der Trennwandsäule (102).

## Revendications

1. Système de colonne à paroi de séparation (100) comprenant :
une colonne à paroi de séparation (102) comprenant une paroi de séparation (106) positionnée dans la section inférieure de la colonne à paroi de séparation (102) pour diviser la section inférieure de la colonne à paroi de séparation (102) en un premier côté (108) et un second côté (109) ;
un premier rebouilleur (110) à l'extérieur de la colonne à paroi de séparation (102) et en communication fluidique avec le premier côté (108) de la section inférieure de la colonne à paroi de séparation (102) ;
un second rebouilleur (112) à l'extérieur de la colonne à paroi de séparation (102) et en communication fluidique avec le second côté (109) de la section inférieure de la colonne à paroi de séparation (102) ;
une pompe à chaleur (118) en communication fluidique avec la colonne à paroi de séparation (102) et le second rebouilleur (112) et configurée pour comprimer une première partie du produit de tête provenant de la colonne à paroi de séparation (102) ;
un second condenseur (114) en communication fluidique avec la colonne à paroi de séparation (102) et configuré pour recevoir une seconde partie du produit de tête provenant de la colonne à paroi de séparation (102) ; et
un premier condenseur (120) en communication fluidique avec le second rebouilleur (112).

2. Système de colonne à paroi de séparation (100) selon la revendication 1, dans lequel le second rebouilleur (112) est couplé fluidiquement à la colonne à paroi de séparation (102) pour alimenter en reflux le second côté (109) de la colonne à paroi de séparation (102) et de préférence également le premier rebouilleur (110) est couplé fluidiquement à la colonne à paroi de séparation (102) pour alimenter en reflux le premier côté (108) de la colonne à paroi de séparation (102).

3. Système de colonne à paroi de séparation (100) selon la revendication 1, comprenant en outre un accumulateur (116) en communication fluidique avec les premier et second condenseurs (120, 114).

4. Système de colonne à paroi de séparation (100) selon la revendication 1 ou 3, dans lequel l'accumulateur (116) est en communication fluidique avec la colonne à paroi de séparation (102) pour alimenter en reflux la colonne à paroi de séparation (102).

5. Procédé de production d'un produit hydrocarboné i-C₄, le procédé comprenant :
l'introduction d'une alimentation contenant des hydrocarbures dans la colonne à paroi de séparation (102) du système de colonne à paroi de séparation (100) selon l'une quelconque des revendications 1 à 4 ;
l'alimentation, à partir du premier côté (108) de la colonne à paroi de séparation (102), du produit de queue comprenant des hydrocarbures C₅₊ dans le premier rebouilleur (110) ;
l'alimentation, à partir du second côté (109) de la colonne à paroi de séparation (102), du produit de queue comprenant des hydrocarbures n-C₄ dans le second rebouilleur (112) ;
l'alimentation d'une première partie du produit de tête provenant de la colonne à paroi de séparation (102) dans la pompe à chaleur (118) et la compression de la première partie du produit de tête dans celle-ci ;
l'alimentation du produit de tête comprimé provenant de la pompe à chaleur 118) dans le second rebouilleur (112) pour échanger de la chaleur entre le produit de tête comprimé et le produit de queue comprenant des hydrocarbures n-C₄, et
l'alimentation du produit de tête comprimé provenant du second rebouilleur (112) dans un premier condenseur (120) et sa condensation dans celui-ci,
dans lequel le système de colonne à paroi de séparation (100) comprend en outre un second condenseur (114) et une seconde partie du produit de tête est alimentée à partir de la colonne à paroi de séparation (102) dans le second condenseur (114).

6. Procédé selon la revendication 5, dans lequel un courant de reflux est alimenté à partir du second rebouilleur (112) dans le second côté (109) de la section de queue de la colonne à paroi de séparation (102).

7. Procédé selon la revendication 5 ou 6, dans lequel un courant de reflux est alimenté à partir du premier rebouilleur (110) dans le premier côté (108) de la section de queue de la colonne à paroi de séparation (102).

8. Procédé selon la revendication 7, dans lequel le système de colonne à paroi de séparation (100) comprend en outre un accumulateur (116) et le produit de tête est alimenté à partir du premier condenseur (120) dans l'accumulateur (116).

9. Procédé selon la revendication 5, comprenant en outre l'alimentation de la seconde partie du produit de tête dans l'accumulateur (116).

10. Procédé selon la revendication 9, comprenant en outre la production d'une charge d'hydrocarbure i-C4 à partir de l'accumulateur (116).

11. Procédé selon la revendication 10, comprenant en outre l'acheminement d'un courant de reflux à partir de l'accumulateur (116) dans la colonne à paroi de séparation (102).
